# EUROPÄISCHE PATENTSCHRIFT

(11) **EP 0 984 718 B1**
(45) Veröffentlichungstag und Bekanntmachung des Hinweises auf die Patenterteilung: **02.01.2003**
(21) Anmeldenummer: 99915532.8
(22) Anmeldetag: 22.02.1999
(51) Int. Cl.: A61B 5/00

(54) **Messgerät zur Ermittlung des Konzentrationsgehaltes von in Flüssigkeit enthaltenen Substanzen mittels einem ATR Element.**
Device for the determination of the concentration of fluid components by using an ATR element
Dispositif de détermination de la concentration des substances dans des liquides à l'aide d'un élément ATR

(30) Priorität: 10.03.1998 DE 29804100 U
(43) Veröffentlichungstag der Anmeldung: 15.03.2000
(73) Patentinhaber: Dittel, Rudolf H., 86343 Königsbrunn (DE)
(72) Erfinder: Dittel, Rudolf H., 86343 Königsbrunn (DE)
(74) Vertreter: Pfister, Helmut, Dipl.-Ing.
(86) Internationale Anmeldenummer: EP9901139
(87) Internationale Veröffentlichungsnummer: WO99044493

(56) Entgegenhaltungen:
- WO-A-93/09421
- CH-A- 612 271
- US-A- 5 246 004
- US-A- 5 370 114
- US-A- 5 379 764
- US-A- 5 694 930

## Beschreibung

### A. Die Zielsetzung des Systems

Die Zielsetzung des neuartigen Bestimmungssystems ist der unmittelbare qualitative und quantitative, quasizeitverzugsfreie, hochspezifische Nachweis von Inhaltstoffen des Blutes durch Messung spektraler Signaturen.

Die zur Anwendung gebrachte Meßmethode gehört zur Kategorie der sogenannten aktiven Verfahren, die dadurch gekennzeichnet sind, daß das Meßobjekt mit Strahlung einer bestimmten Eigenschaftsausprägung direkt oder indirekt beaufschlagt wird und aus den Eigenschaften der zurückkehrenden Strahlungsanteile auf das Vorhandensein bestimmter Inhaltsstoffe in einer Flüssigkeit und deren Konzentration geschlossen wird.

Bei dem Erfindungsgegenstand handelt es sich um ein Gerät, bei dem die Meßvorrichtung mit der die nachzuweisenden Substanzen enthaltenden Flüssigkeit nicht in Berührung gebracht wird: Die Bestimmung des Vorhandenseins und der Konzentration kann durch eine undurchsichtige Trennwand, z. B. auch durch das Hautgewebe, hindurch erfolgen. (Am Meßverfahren ändert sich nichts, wenn die Meßvorrichtung mit der Flüssigkeit in Berührung gebracht werden kann. In diesem Fall treten die sonst von der Trennwand zwischen Flüssigkeit und Meßapparatur ausgehenden Einflüsse jedoch zwangsläufig nicht auf).

### B. Stand der Technik

Es existieren eine Reihe von Anwendungsbereichen, bei denen die Bestimmung chemischer Substanzen in einer wässrigen Lösung erforderlich ist, ohne daß hierzu eine Flüssigkeitsprobe entnommen wird oder die Flüssigkeit mit den Meßsensor in Kontakt gebracht werden kann.

Ein Beispiel von anwendungsspezifischer Bedeutung stellt die Ermittlung von metabolischen (und anderen) Inhaltsstoffen im Blut durch die Haut hindurch ohne vorangehende Blutentnahme, Verletzung der Haut oder Einbringung/Implantation eines den Meßvorgang unterstützenden Elements dar. Bei den Meßobjekten kann es sich um natürliche physiologische Substanzen (wie z. B. Blutzucker, Cholesterin, Lipide, Peptide, Harnstoff und weitere) oder um, wie auch immer dem Blut zugeführte nicht-metabolische Substanzen (wie z. B. Alkohol, Medikamente, und weitere) handeln.

Die bisher angewandten Methoden zur Bestimmung von Inhaltsstoffen des Blutes, z. B. Blutzucker, Alkohol und anderen, erfordern die Entnahme einer Blutprobe, verbunden mit einer gewissen Wahrscheinlichkeit der Infizierung, einem erheblichen naßchemischen und meßtechnisch-apparativen Aufwand, sie sind arbeitsintensiv und langwierig und sie können zur Gewährleistung einer gewissen Zuverlässigkeit und Wirtschaftlichkeit zumeist nur in besonders ausgestatteten Laboratorien durchgeführt werden.

Eine Bestimmung der Blutinhaltsstoffe in beliebig häufiger Folge und zu frei wählbaren Zeitpunkten durch den Probanden selbst ist mit Geräten des gegenwärtigen Stands der Technik weitgehend ausgeschlossen.

Im folgenden wird der aktuelle Stand der Technik auf der Grundlage gewerblich angebotener Geräte und erteilter Patente dargelegt.

Grundsätzlich wird zwischen invasiven und nicht-invasiven Verfahrensweisen unterschieden.

Aus Gründen der Zweckmäßigkeit wird nicht der gesamte Umfang der Geräte und Verfahren der genannten Kategorien aufgeführt, sondern nur solche, die der Erfindungsidee bezüglich ihrer Methodik nahe kommen. Ausgewählt wurden hierzu ausschließlich Einsatzfälle zur Bestimmung der Blutzuckerkonzentration.

### B.1 Geräte mit minimal-invasiver Verfahrensweise

### Anbieter: CYGNUS Inc

Interstitialflüssigkeit (ISF) wird durch Elektrophorese-effekt mit Hilfe eines Heftpflasters durch die Haut hindurch extrahiert. Das Heftpflaster befindet sich auf der Rückseite einer Auflage mit der Form und in der Größe einer Armbanduhr; Infektionsgefahr; Heftpflaster ist über eine Dauer von 12 Stunden verwendbar;
* Glucosebestimmung ex-vivo indirekt durch ISF-Analyse;

### Anbieter: Biojet Inc

Verfahren wie bei CYGNUS Inc, jedoch zusätzliche Verwendung einer ISF-Mikrosonde; Kombination mit Meßgerät; Infektionsgefahr;
* Glucosebestimmung ex-vivo indirekt durch ISF-Analyse;

### Anbieter: Technical Chemical Products

ISF wird wie bei CYGNUS extrahiert; lange Meßzeiten;
* Glucosebestimmung erfolgt "elektronisch" ex-vivo;

### Anbieter: Inteq Inc

ISF wird mit Mikrolanzette abgesaugt; grundsätzliche zeitliche Verzögerung der Glucosewertausprägung in der ISF zu jener im Blut beträgt zwischen etwa 15 und etwa 25 Minuten;
* Glucoebestimmung durch spektroskopische Analyse im Infrarotbereich;

### Anbieter: Minimed Inc

Implantierter Teflon-Katheter zur Entnahme von ISF; Infektionsgefahr; täglicher Wechsel des Plazierungsortes für Katheter erforderlich;
* Glucosebestimmung ex-vivo über ISF-Analyse;

### Anbieter: Synthetic Blood International

Implantierte Meßsonde; Reduktion von Hydrogenperoxid verursacht Elektronenfluß; elektrisches Signal steht in Bezug zum Konzentrationswert des Blutsauerstoffs; die Korrelation Sauerstoff/Glucose unsicher; zudem Abnahme der Meßgenauigkeit durch Belegung der Elekrodenoberfläche mit Zellen und Proteinen;
* Glucosebestimmung ex-vivo durch digitale Auswertung;

### Anbieter: SpectR Inc

ISF-Extraktion durch Mikropore mit Übertragung auf Teststreifen;
* Glucosebestimmung ex-vivo mit Hilfe des Teststreifens;

### Verfahren, ohne Anbieter: Glucose-Sensor

Basiert auf Verhältnis der Konzentration von Natrium in der entnommenen ISF (korreliert mit elektrischer Leitfähigkeit) und der interstitialen Natriumkonzentration, die als konstant angenommen wird; angeblich keine Eichung erforderlich; die Nadel zur Entnahme der ISF muß mindestens ein Mal pro Tag ausgetauscht werden;

### B.2 Geräte mit nicht-invasiver Verfahrensweise

### Anbieter: Futurex Inc

Angeblich Messung der elektro-magnetischen Aura des Probanden; wissenschaftlicher Bezug nicht erkennbar;
* Glucosebestimmung auf "esoterische Art";

### Anbieter: Peti Tech Inc

Bestrahlung der Retina; Bezug zum Glucosewert nicht nachvollziehbar;
* Glucosebestimmung visuell; Korrelation zwischen Meßwert und Glucosewert unsicher;

### Anbieter: Sensor for Medicine Inc

Fluoreszensmessung der Moleküle von Blutinhaltsstoffen;
* Glucosebestimmung indirekt über Strahlungsintensität; Korrelation von Strahlungsintensität/Glucosewert unsicher;

### Anbieter: CME Telemetrix Inc

Durchleuchtung der Fingerkuppe mit Infrarotstrahlung; großer gerätetechnischer Aufwand;
* Glucosebestimmung angeblich durch Messung der spektralen Signatur;

### Anbieter: Biocontrol Technology Inc

Bestrahlung des gesamten Unterarms; großer gerätetechnischer Aufwand; mehrstündige Eichung erforderlich pro Patient;
* Glucosebestimmung angeblich durch Messung der spektralen Signatur;

### Verfahren, ohne Anbieter: Methode Strahlungsstreuung

Streuung von Licht bei der Durchleuchtung der Haut bei Wechselwirkung mit Glucosemolekülen; Meßwert abhängig von Glucosekonzentration; großer gerätetechnischer Aufwand;
* Glucosebestimmung über Indizes der Brechung und der diffusen Reflexion; durch Abhängigkeit von Glucosekonzentration und Eindringtiefe ergibt sich Mehrdeutigkeit des Signalwertes zur Glucosebestimmung;

### B.3 Patente

US-5 694 930 offenbart ein Messgerät gemäß Oberbegriff des anspruchs 1.

### Patent CH 612271: Kaiser

Die Absorptionswerte des Blutes des Probanden in einer Meßzelle werden gleichzeitig mit jenen in einer Vergleichslösung in einer Referenzzelle bestimmt. Die Differenz der Signalwerte ist proportional zur vorliegenden Glucose-Konzentration im Probandenblut. Strahlungsquelle ist ein CO₂-Laser im nahen Infrarot-Bereich des elektromagnetischen Spektrums. Die Wellenlängen sind so festgelegt, daß eine Erwärmung des Blutes während des Meßvorgngs eintreten und damit eine Verfälschung der Meßwerte erfolgen kann.

Das Problem der Gewebeabsorption und der Einfluß anderer Inhaltsstoffe des Blutes auf den Gesamtmeßwert sowie die Diskriminierung des Glucoseanteils wird nicht erklärt.

### Patent US 3958560: March

### Patent US 5009230: Hutchinson

Diesen Patenten liegt die Nutzung von Polarisationseffekten zur nicht-invasiven Bestimmung des Konzentrationswertes von Blutzucker zugrunde. Bei dem Verfahren nach March erfolgt die Messung durch Bestrahlung des Auges, bei der Methode nach Hutchinson direkt an der Blutflüssigkeit.

Zur Messung wird Strahlung mit Wellenlängen zwischen 0,94 µm bis 1 µm verwendet. Als wesentlicher Nachteil erweist sich die Tatsache, daß in diesem Spektralbereich eine nur minimale Absorption durch Glucoseanteile in wässrigen Lösungen erfolgt und daher das Meßsignal nur unspezifisch ausgeprägt ist.

### Patent US 4655225: Dähne

Diesem Patent liegt ein spektrophotometrisches Verfahren zugrunde. Dabei werden die Signalwerte verschiedener Spektralbänder im Wellenlängenbereich zwischen 1,0 µm bis 2,5 µm zur Bestimmung der Blutzuckerkonzentration kombiniert bewertet. Diese Methodik eliminiert zu einem gewissen Grade den degradierenden Einfluß der Gewebeabsorption auf die Meßwertgenauigkeit und -zuverlässigkeit. Durch diese kombinatorische Werteermittlung werden hohe Energien der Meßstrahlung überflüssig und damit eine Erwärmung des Meßobjekts vermieden.

### Patent US 5137023: Mendelsohn

Diesem Patent zur Bestimmung der Glucosewerte im nahen Infrarotbereich liegt die Verwendung eines pulsativen Photolethysmographen zugrunde. Dabei wird sowohl das Transmissions- als auch das Reflektionsverhalten der Meßstrahlung bewertet. Zur Bestimmung der Blutzuckerkonzentration wird jeweils die Differenz bzw. das Verhältnis der Meßsignalwerte für zwei verschiedene Wellenlängen verwendet. Damit werden auch die Werteunsicherheiten infolge der Gewebeeinflüsse vermindert.

Als Signalträger wird hier (wie auch beim Patent US 4655225) Strahlung mit einer Wellenlänge kleiner als etwa 2,5 µm verwendet. In diesem Spektralbereich erfolgt eine nur geringe Beeinflussung der Strahlungssignatur durch Wechselwirkung mit Blutzucker.

Dadurch ergibt sich eine sehr schwache glucosespezifische Signalausprägung. Insbesondere wird damit eine Diskriminierung zu Signaleinflüssen anderer Blutbestandteile erschwert.

### Patent WO 81/99622: Mueller

Die Konzentration von Stoffwechselprodukten im Blut erfolgt durch spektroskopische Messungen in fernen Infrarotbereich, bei Wellenlängen um 10 µm. Hierzu wird zunächst die Absorption bei einer Wellenlänge von 9,1 µm bestimmt. Zur Absicherung dieses Meßwertes wird zusätzlich ein Meßwert bei einer Wellenlänge von 10,5 µm aufgenommen.

Das Problem der Meßwertbeeinflussung durch Gewebe wird nicht erklärt. Auch erfolgt keine Berücksichtigung unterschiedlicher Meßwertausprägungen in der systolischen und diastolischen Phase.

### Patent US 5028787: Rosenthal

Die Glucosebestimmung erfolgt im nahen Infrarot-Bereich bei Wellenlängen zwischen 0,5 µm bis 1,1 µm. In diesem Spektralbereich weist Blutzucker keine primäre Absortionsbänder auf, sodaß nur eine verhältnismäßig schwache glucosespezifische Signalsignatur zu erwarten ist.

### C. Vorteile eines Geräts zur nicht-invasiven Feststellung von Inhaltsstoffen in einer Flüssigkeit in quasi Echtzeit

Die Anforderungen an die Leistungsfähigkeit eines Meßgerätes zur nicht-invasiven Feststellung von Inhaltsstoffen in einer Flüssigkeit in quasi Echtzeit wird am Beispiel der Blutzuckerbestimmung dargelegt. Das dafür zur Anwendung gebrachte Meßprinzip ist jedoch auch für erweiterte Aufgabenstellungen im nichtmedizinischen Bereich einsetzbar. Es gewinnt besondere Bedeutung wegen des möglichen Nachweises von Substanzen geringer Konzentration durch eine Trennwand hindurch infolge einer provozierten charakteristischen Indikatorreaktion.

Die bisher angewendeten und in Patenten dargelegten Verfahren zur Blutzuckerbestimmung erlauben keine kontinuierliche und auch keine beliebig häufige Ermittlung der Blutzuckerwerte zu frei wählbaren Zeitpunkten. Dadurch ergibt sich im allgemeinen ein unvollständiges und wenig schlüssiges Bild zur Tageshistorie der Blutzuckerkonzentration. Insbesondere können kurzzeitige Schwankungen der Glucosewerte übersehen werden.

Häufig und zu beliebigen Zeitpunkten gewonnene Blutzuckerwerte würden für die Behandlung der verschiedenen Diabetes-Erkrankungen den Vorteil bieten, daß Gegenmaßnahmen, wie z. B. die Verabreichung von Insulin bei Diabetes Typ I oder von Medikamenten bei Diabetes Typ II, gezielt dann vorgenommen können, wenn ein meßtechnisch nachgewiesener Bedarf tatsächlich besteht. Im Gegensatz hierzu erfolgen die Gegenmaßnahmen bisher vorsorglich in einem festgelegten Zeitrahmen oder bei eintretendem Unwohlsein.

Eine beliebig häufig wiederholbare nicht-invasive Ermittlung der Blutzuckerwerte ergäbe auch für den Fall einer Schwangerschafts-Diabetes größere Sicherheit bei der Verfolgung des Werteverlaufes und damit im Bedarfsfall die Möglichkeit von zielgerichteten und damit den Schwangerschaftsvorgang nicht unnötig belastenden medikamentösen Maßnahmen.

Die derzeit zur Anwendung gelangenden Verabreichungsmethoden für Insulin werden von den Probanden als weit weniger störend empfunden als eine häufige Kontrolle des Blutzuckerwertes, z. B. durch Entnahme von Blut aus der Fingerkuppe.

Jederzeit ohne Blutentnahme feststellbare Blutzuckerwerte würden auch die Durchführung von umfassenden und systematischen Vorsorgeuntersuchungen über einen längere Zeitspanne vereinfachen unter vorgegebenen Randbedingungen, wie z. B. die Belastung des Probanden, die Zeitpunkte der Nahrungsaufnahme, die Art und Menge der Nahrung, usw. Durch solche systematischen Vorsorgeuntersuchungen können akute Fälle der sogenannten Impaired Glucose Tolerance IGT (= gestörte Glucose-Toleranz) aufgedeckt und damit eine bestehende Neigung zur chronischer Diabetes bereits im Vorfeld entdeckt und damit gezielt behandelt werden.

Die verallgemeinerte Erweiterung eines nicht-invasiven Meßverfahren mit Verfügbarkeit der Meßwerte in Echtzeit bietet auch für andere Anwendungsgebiete mit medizinischen Bezug neue Perspektiven. So ermöglichst es die Feststellung der Alkoholkonzentration im Blut in weniger als einer Minute ohne daß hierzu eine Blutprobe entnommen oder zur Werteermittlung ein Labor in Anspruch genommen werden muß.

Im Umweltbereich ist dieses Verfahren auch für die analytische Feststellung von Herbiziden und Insektiziden und Kohlenwasserstoffen in Trinkwasser geeignet. Hierbei kann der Meßsensor allerdings mit der Flüssigkeit direkt in Kontakt gebracht werden.

Der wesentliche Neuheitswert des Meßverfahrens besteht jedoch in seiner Eigenheit, die Inhaltsstoffe wässriger Lösungen durch eine Begrenzungsschicht hindurch quantitativ und qualitativ feststellen zu können. Das Verfahren kann deshalb für ex-vivo-, in-vivo- und invitro-Untersuchungen angewandt werden.

Eine Selektions- bzw. Verstärkungseffekt des Meßsignals kann durch besondere Maßnahmen erreicht werden. So kann z. B. eine reaktive Substanz in die Flüssigkeit (z. B. das Blut) eingebracht werden und diese Substanz durch die Meßstrahlung oder eine andere Strahlungsart zu einer Reaktion angeregt werden. Diese Reaktion kann zu einer vorübergehenden oder bleibenden Änderung der Signatur der von der Flüssigkeit und/oder deren Inhaltssubstanzen emittierten Strahlung führen, je nachdem, ob eine vorübergehende oder eine bleibende physikalisch-chemische Veränderung der Substanz-Wechselwirkung durch die Strahlung erzeugt wird.

Dieses Verfahren kann z. B. in Form einer Indikatorreaktion zum Nachweis von Substanzen extrem niederer Konzentration in Flüssigkeiten und damit das Vorliegen eines schwachen Rückstreusignals dahingehend genutzt werden, daß durch die provozierte Reaktion mit einem zu diesem Zweck eingebrachten spezifischen Reaktionsstoff über den stochastischen oder systematischen Störpegel liegende Signalausprägungen erzeugt werden. Damit ist das Vorhandensein einer Substanz nachweisbar, die meßtechnisch sonst nicht genügend signifkant in Erscheinung treten würde. Solche provozierten Ausprägungen des Meßsignals sind z. B. für Antikörper- oder hormonale Reaktionen charakteristisch und können damit zum Nachweis von Krankheitserregern sowie von molekularen Wechselwirkungen und Konzentrationsänderungen von Hormonen im Blut verwendet werden.

Grundsätzlich kann in Erweiterung dieser Methode auch die dem Sensor zugewandte Außenseite der Trennwand oder die Sensoraußenfläche mit einer aktiven Substanz bestrichen werden, die zu einer selektiven Minderung bzw. Verstärkung und damit zu einer signifikanten Ausprägung der zum Nachweis einer Substanz bzw. einer Reaktion erforderlichen charakteristischen Strahlungssignatur führt.

### D. Charakteristika eines Geräts zur nicht-invasiven Feststellung von Inhaltsstoffen in einer Flüssigkeit in quasi-Echtzeit

(Die Darstellung erfolgt bevorzugt am Beispiel der Blutzuckerbestimmung).

Ein Gerät zur Bestimmung des Blutzuckerwertes muß folgende Anforderungen erfüllen:
* es sollte eine Messung zu beliebigen Zeitpunkten beliebig häufig erlauben, ohne daß hierzu Blutproben entnommen werden müssen, d. h. es muß eine nicht-invasive Meß-Methode zugrunde gelegt werden;
* es muß eine Auswertung der Einzel- und Massenmeßwerte (Werte-Historie) erlauben, um damit für eine bestimmte Ausprägung der Glucosewerte unter Berücksichtigung der Blutzucker-Historie zu probandenspezifischen Maßnahmeempfehlungen zu gelangen.

### Wellenlängenbereiche der Meßstrahlung

Die Nicht-Invasivität wird am besten durch Verwendung von Strahlung einer sich als ungefährlich erwiesenen Wellenlänge und Energiedichte erreicht. Als geeignet hat sich dabei die Infrarotstrahlung erwiesen, die einen Wellenlängenbereich von etwa 0,8 µm bis etwa 100 µm aufweist. Dieser Wellenlängenumfang wird zumeist unterteilt in den Bereich des nahen Infrarot (NIR) von etwa 0,8 µm bis etwa 2,5 µm Wellenlänge, des mittleren Infrarot (MIR) von etwa 2,5 µm bis etwa 15 µm Wellenlänge und des fernen Infrarot von etwa 15 µm bis etwa 100 µm Wellenlänge. (Statt der Wellenlänge wird auch die Wellenzahl = 1/Wellenlänge (in cm) bzw. 10⁴/Wellenlänge (in µm) angegeben.

Strahlung durchdringt für Wellenlängen des sogenannten Infrarotbereichs die Haut in unterschiedlichem Maße. Bei niederen Frequenzen des nahen Infrarot wird die Strahlung bevorzugt durch Blut oder Hautpigmente, bei höheren Frequenzen vor allem durch Wasser absorbiert.

Die charakteristischen Spektralsignaturen für einzelne Substanzen liegen in bestimmten Abschnitten des genannten Infrarotbereichs. Die Tabelle 1 enthält die typischen Wellenzahlen zur spektroskopschen Identifizerung einiger biologisch relevanter Substanzen.

C-C-, C-N-, C-O- und andere einfache Kohlenstoffverbindungen besitzen charackteristische Absorptionswellenlängen im Bereich zwischen 6,5 µm bis 15 µm. Da Glucose diese Art von Bindungen ebenfalls enthält, weist sie in diesem Spektralbereich ausgeprägte Spektralsignaturen auf.

Da jedoch im Blut auch andere Substanzen vorhanden sind, die einerseits in einem Meßvorgang mitbestimmt werden können, andererseits deren charakteristische Spektralsignatur zur Unterscheidung gegenüber Blutzucker und auch untereinander heranzuziehen ist, wird ein breiter angelegter Infrarotbereich für die Messung herangezogen, als er für die Bestimmung nur einer einzelnen Substanz unbedingt erforderlich ist. Lediglich für spezielle Meßaufgaben, wie z. B. die Ermittlung von Alkohol im Blut ist eine Beschränkung auf einen begrenzten Wellenlängenbereich möglich, um solche im Blut in natürlicher Ausprägung nicht vorhandene Substanzen zu erfassen und zu quantifizieren.

Bewährt hat sich als Meß- und Auswertemethode hierbei das Multispektralprinzip. Dabei nutzt man die substanzspezifischen Absorptions- bzw. ReflexionsEigenschaften. Mehrdeutigkeiten von Signaturausprägungen einer Substanz infolge partieller Überlagerungen mit den Signaturausprägungen anderer Substanzen lassen sich über eine multispektrale Bewertung der Einzelausprägungen aufklären.

In seiner universell einsetzbaren Version wird das Gerät für eine Meßstrahlung im nahen, mittleren und fernen Infrarotbereich, beginnend ab etwa 0,8 µm bis etwa 30 µm ausgelegt. Je nach Meßaufgabe werden davon nur ausgewählte Teilbereiche des Spektrums herangezogen. Bei Spezialausführung des Gerätes z. B. zur Bestimmung nur einer oder mehrerer ähnlicher Substanzen, wird der Wellenlängenumfang auf den erforderlichen Meßbereich beschränkt ausgelegt.

### Strahlungsquellen

Die verwendeten Strahlungsquellen können sowohl kohärenter (= Laser) als auch inkohärenter Natur sein.

Eine Laser-Strahlungsquelle bietete den Vorteil hoher Energiedichte und hoher Wellenlängenkonstanz. Sie verlangt jedoch zur Bestimmung spezifischer Substanzen, die auch eine Unterscheidung zu anderen Substanzen beinhaltet, eine Veränderung der Strahlungsfrequenz über einen bestimmten Frequenzbereich. Bei den derzeit verfügbaren Laser-Strahlungsquellen ist diese Durchstimmbarkeit begrenzt. Aus diesem Grunde ist zur Erzeugung von Strahlung über einen größeren Frequenzbereich die Verwendung mehrerer Laser-Strahlungsquellen erforderlich.

Bei einer, auf eine einzige Substanz ausgelegten Gerätekonfiguration, wie z. B. zur Bestimmung der Alkoholkonzentration in Blut, ist eine einzige angepaßte Laser-Strahlungsquelle zum Zweck der Bestimmung und Diskriminierung ausreichend. Die Verwendung von Laser-Strahlungsquellen führt auch zu Problemen bei der Miniaturisierung, sodaß beim gegenwärtigen Stand der Technik deren Einsatz bevorzugt für (stationäre) Laborgeräte in Betracht kommt. Für "Handgeräte" sind sie nur dann geeignet, wenn die Meßaufgabe mit (bevorzugt) einer oder weniger Laser-Strahlungsquellen durchführbar ist.

Die inkohärenten Strahlungsquellen (wie z. B. eine Nernst-Strahler) besitzen den Vorteil, Wellenlängen über einen großen Bereich des gesamten Infrarotspektrums zu emittieren. Die Durchführung des Meßvorgangs erfordert jedoch eine Frequenzbegrenzung (= Bandpaßfilterung), wobei das Paßband (bei möglichst konstant bleibender Frequenzbandbreite) über den erforderlichen Wellenlängenbereich verschoben wird.

Bei Verwendung einer inkohärenten Strahlungsquelle ist die Verwendung von Bandpaßfiltern (eine geeigneten technischen Ausführung in Abhängigkeit von der besonderen Aufgabenstellung) zwingend erforderlich.

Im allgemeinen zeigen inkohärente Strahlungsquellen zudem ausgeprägte wellenlängen- und temperaturspezifische Abhängigkeit der emittierten Strahlungsenergie. Aus diesem Grunde sind bei Verwendung inkohärenter Strahlungsquellen technische Maßnahmen zur Gewährleistung einer konstanten frequenzabhängigen Energiedichte über den Meßfrequenzberich und für die vorgegebenen Bandpaßbreite des Filters erforderlich.

Eine frequenzabhängige Energievariation ist auch erforderlich, um über den gesamten Meßbereich der Messung konstante Energiedichte für die jeweilige Eindringtiefe zu gewährleisten, da die Eindringtiefe grundsätzlich von der Frequenz der Meßstrahlung bestimmt wird.

Weitere Maßnahmen der Manipulation der Meßstrahlung werden unter "Strahlungsmodulation" dargelegt.

### Strahlungsführung am Meßort

Zum Zweck der Bestimmung der Blutinhaltsstoffe wird eine geeignete Stelle des menschlichen Körpers mit Meßstrahlung beaufschlagt, die aus dem Körper durch Streuung und/oder Reflektion zurückkehrende modulierte Strahlung erfaßt und bezüglich ihrer geänderten spektralen Signatur analysiert.

Auf dem Weg von der Strahlungsquelle zum Meßsensor erfolgt eine Abschwächung der Strahlungsintensität und gleichzeitig eine Überlagerung der substanzspezifischen Signalanteile durch stochastische und systematische Einwirkungen.

Die Unzulänglichkeit eines schwachen Meßsignals kann behoben werden durch eine optische Vorrichtung, die ein Eindringen der gesamten Strahlung in tiefere Schichten der Trennwand (= Hautschichten) und deren Totalreflektion ohne Energieverlust gewährleistet. Es handelt sich hierbei um eine sogenanntes ATR-Element (ATR= attenuated total reflection = abschwächte Totalreflektion).

Das ATR-Element kann in verschiedenen zweckdienlichen Ausführungen zur Anwendung gelangen. Wesentlich bei dessen konstruktiver Gestaltung ist dabei die Sicherstellung einer schlüssigen Auflage am Meßort, zudem eine geeignete Strahlführung zur Gewährleistung einer genügend tiefen Eindringung des Meßstrahls bei maximal häufiger Wechselwirkung mit der Meßsubstanz sowie der Totalreflektion.

Ein solches ATR-Element kann auch am Ende von mindestens zwei Lichtwellenleitern angebracht werden, wobei die Meßstrahlung in einem Leiter zu ATR-Element hin und im zweiten Leiter vom ATR-Element weg zum Sensor hin geleitet wird.

Eine weitere technische Realisierung des ATR-Prinzips ergibt sich durch die Verwendung kristalliner Fasern, die Infrarotstrahlung leiten. Der Vorteil bei Verwendung dieses Materials besteht in dessen sehr hoher Transparenz im Spektralbereich von 0,4 µm bis 30 µm.

Zudem weist es eine geringere Wasserlöslichkeit auf und ist resistenter gegenüber Oberflächendegradation als die üblicherweise verwendeten Materialien wie Si, GaAs und einige Zn-Verbindungen.

Wegen der guten Bearbeitbarkeit des kristallinen Materials können Oberflächenrauhigkeiten kleiner als 50 nm und Abweichungen von der Idealform in der Größenordnung von 10 µm erreicht werden. Wegen seiner hohen Widerstandsfähigkeit gegenüber aggressiven Flüssigkeiten und Gasen eignet sich dieses Material auch für den Meßeinsatz in der Umwelttechnik.

Durch ein hyperbolisch geformtes Faserende kann sowohl sichtbare als auch Infrarot-Strahlung über die Faser geleitet und fokusiert und damit höhere Strahlungsintensitäten zum Ort der zu bestimmenden Substanzen übertragen werden. Verzweigt man den Strahlweg auf mehrere Fasern oder auf mehrere aufgespaltene Faserenden, dann können gleichzeitig mehrere benachbarte oder weiter auseinander liegende Meßorte erreicht und bedient werden. Für den Fall der Blutmessung können dies z. B. mehrere Aufpunkte auf die Lippe und/oder auf der Zunge sein. Dadurch werden Meßfehler durch zufällige Wahl eines einzigen, jedoch ungeeigneten Auflageortes vermieden.

Wegen der speziellen optischen Eigenschaften des ATR-Elements wird wegen der Totalreflektion der eingebrachten Strahlungsenergie der relative Einfluß stochastisch einwirkender Störeinflüsse auf das Meßsignal gering gehalten. Somit erfolgen die wesentlichen Veränderungen der spektralen Strahlungsintensität durch Absorption der mit der Strahlung in Wechselwirkung tretenden Inhaltsstoffe des Blutes.

Ein Nachteil der konventionellen ATR-Anwendung besteht in deren geringen Sensitivität zur Erfassung von Substanzen mit geringer Konzentration. Dies ist bedingt durch die Notwendigkeit, den Meßstrahl unter einem bestimmten Winkel über eine abgeschrägte Kante in das ATR-Element einzubringen, um Totalreflektion der Strahlung zu gewährleisten. Dadurch werden, bedingt durch die interne Reflektion im ATR-Element entlang der Auflagefläche nur Meßpunkte in inkrementalen Abständen erfaßt, die mindestens der geometrischen Strahlweite entsprechen. Als Folge davon tritt der Strahl mit einem bestimmten Teilsegment (minimaler Ausdehnung) nur jeweils einmalig in Wechselwirkung; die Bereiche zwischen diesen Aufpunkten werden nicht meßtechnisch erfaßt.

Durch eine flächendeckende Eindringung des Meßstrahls über die gesamte Auflagefläche des Kontaktkörpers und die damit gleichzeitig stattfindende Mehrfacherfassung jeden Meßpunktes würde erreicht, Substanzen geringer Auftretenshäufigkeit zu detektieren und zudem die Meßgenauigkeit grundsätzlich zu erhöhen. Die Folge wäre neben dem Nachweis ansonsten nicht entdeckbarer Substanzen auch die signifikante Unterscheidbarkeit von Stoffen mit ähnlich ausgeprägter spektraler Signatur.

Die Verbesserung der Erfaßbarkeit von Substanzen geringer Konzentration wird durch eine optische Vorrichtung erreicht, mit deren Hilfe die Meßstrahlung in einen optischen Resonanzzustand versetzt wird. Man bedient sich hierzu einer Schichtung von optischen Materialien mit verschiedenen Brechnungskoeffizienten, die in Abhängigkeit von der Strahlungsfrequenz und dem repräsentativen Brechungskoeffizienten des Meßobjekts - das ist im vorliegenden Fall das Gewebe und die blutführenden Bereiche - einen bestimmten räumlichen Abstand zueinander aufweisen müssen.

Im Prinzip kann diese Vorrichtung aus einer Halbkugel bestehen, an deren ebenen Fläche zwei aufeinanderfolgende transparente Schichten schlüssig aufgebracht sind. Die äußere Schicht stellt dabei auch gleichzeitig die Auflagefläche (= Kontaktfläche) auf die Hautoberfläche dar.

Um den erforderlichen Resonanzustand zu erreichen, müssen das Material der Halbkugel und das der äußeren Resonanzschicht einen hohen Brechungsindex n₁ bzw n₃, das der dazwischenliegenden Abstandsschicht einen niedrigen Brechungsindex n₂ aufweisen. Dabei kann n₁ = n₃ sein und damit wird n₂ < n₁, n₂ < n₃.

Die Dimensionierung der Halbkugel, die Dicke der Abstandsschicht und der Resonanzschicht wird in Abhängigkeit von der Frequenz der Meßstrahlung, sowie der optischen Eigenschaften des Meßobjekts festgelegt.

Der Meßstrahl wird über die Halbkugel in das optische Element eingeleitet und kehrt in substanzspezifisch modulierter Form von dort auch wieder zurück. Die Verwendung einer Halbkugel anstelle z. B. eines Prismas bietet den Vorteil, daß der Einstrahlwinkel der Meßstrahlung einfacher variiert werden kann. Dabei ist auch zu beachten, daß die Verhältnisse beim Übergang von der Resonanzschicht zur Hautoberfläche den Anforderungen zur Erzielung von Totalreflektion genügen.

Beim Eintreten des Resonanzfalls bilden sich in der Phasenschicht stehende Wellenstrukturen der Meßstrahlung aus, deren Feldausprägung sich auch in das Meßobjekt hinein auswirken. Da die Resonanzschicht im Vergleich zur Breite der Meßstrahls eine geringe geometrische Ausdehnung aufweist, ergeben sich auch kleine Abstandsinkrements für die Positionen der einzelnen Meßpunkte entlang der Kontaktfläche und deswegen auch eine erhöhte Anzahl von quasi kontinuierlich aneinandergereihten Meßpunkten im Kontaktbereich.

Die im Resonanzfall durch die stehenden Wellenstrukturen erreichten Mehrfachmessungen führen zu einer Betonung der Absorptionsausprägung, damit zu einer Erhöhung der Meßempfindlichkeit und somit gleichzeitig zu einer erhöhten Wahrscheinlichkeit der Detektion von Substanzen geringer Konzentraion im Blut, die mit der herkömmlichen ATR-Anwendung nicht erfaßt würden.

### Polarisation und Pulsung

Für eine vorgegebene Wellenlänge ist der Grad der Reflexion vom Polarisationswinkel der Strahlung abhängig. Durch Wahl eines geeigneten Polarisationswinkels ist es möglich, den Kontrast der spektralen Ausprägung und damit die Wirksamkeit der Detektion und der Unterscheidung von unterschiedlichen Substanzen wesentlich zu erhöhen.

Grundsätzlich ist auch die Verwendung von zufallspolarisierter Meßstrahlung zulässig. Durch die meßtechnisch bedingten Reflexionen der Strahlung an und in den optischen Elementen wird einem Teil der Strahlung jedoch eine bestimmte Polarisationsausprägung aufgezwungen. Da der entsprechende Strahlungsanteil und seine bevorzugte Polarisationsebene sich stark zufallsbedingt einstellen und für den Meßvorgang exakt auch nicht bekannt sind, ergibt sich somit eine gewisse Unsicherheit bei der Erfassung bzw. bei der Interpretation der Meßwerte.

Zur Vermeidung dieser Unsicherheiten wird polarisierte Strahlung - das ist Strahlung einer einheitlichen Polarisationsausprägung - verwendet.

Die Polarisationsebene wird dabei im Verlauf der Messung über den Gesamtbereich aller Polarisationsebenen geändert, wobei jedoch die Horizontal- und der Vertikalpolarisation besondere Beachtung findet. Zudem können auch noch dynamische Variationen vorgenommen werden, wie z. B. zirkular und elliptische Polarisation, und links- und rechtsdrehende Polarisation.

Die geeignete Variation des Polarisationswinkels während des Meßvorgangs führt auch zu einer Erhöhung der Meßgüte im Hinblick auf die Identifizierbarkeit und Unterscheidbarkeit der einzelnen Inhaltssubstanzen.

Substanzspezifische elektrische Feldkomponenten existieren für alle drei räumlichen Richtungen der reflektierenden Strahlung. Durch die Kombination des Reflexionsgrades für verschiedene Polarisationsebenen der Meßstrahlung lassen sich die Absorptionskoeffizienten für alle drei räumlichen Richtungen einer anisotropen Meßprobe bestimmen, ohne daß diese jeweils entsprechend ausgerichtet werden muß. Damit erhält man neben der wellenlängenabhängigen Signatur unter Verwendung zufallspolarisierter Strahlung auch noch einen Satz polarisationsabhängiger Meßvariablen und damit erhöhte Zuverlässigkeit bei der Erkennung einzelner Substanzen und der Festlegung der dazugehörigen Konzentrationen.

Im allgemeinen erfolgt die Strahlungsbeaufschlagung des Meßobjekts kontinuierlich während des geamten Meßvorgangs. In jedem Fall baut sich bei der Einwirkung des Strahlungsfeldes im Meßobjekt ein Gegenfeld auf, dessen Stärke die effektive Eindringtiefe bestimmt. Eine zeitliche Verkürzung der Einwirkdauer der Meßstrahlung führt zu einer größeren Eindringtiefe unter der Voraussetzung, daß während der Einwirkdauer auch ausreichend Strahlungsenergie bereitgestellt wird. Die maximale Eindringtiefe ergibt sich somit durch eine Optimierung des Produkts aus der Strahlungsenergie pro Flächeneinheit bzw. pro Volumeneinheit mit der Zeitdauer der Einwirkung.

Für die Durchführung der Messung bedeutet eine diskontinuierliche Strahlungsbeaufschlagung des Meßobjekts eine erhöhte Eindringtiefe des Meßstrahls. Grundsätzlich könnte dieser Effekt durch Pulsung der Strahlungsenergie erreicht werden. Wegen der Steilheit der Impulsflanke können sich dabei jedoch unerwünschte Resonanzerscheinungen und unerwünschte Modulationsfrequenzen einstellen, sodaß einem stetigen Übergang zwischen Intensitätsminimum und Intensitätsmaximum, z. B. in Sinusform oder zumindest unter Vermeidung von Intensitätsunstetigkeiten, der Vorzug gegeben wird.

### Verbesserung der Spektralsignaturen

Im allgemeinen ist zur Feststellung und zur Identifizierung einer bestimmten Substanz in einer Stoffmischung die Detektion der spektralen Signaturen über einen größeren Wellenlängenbereich notwendig als dies bei isoliertem Vorliegen nur einer einzigen Substanz erforderlich ist. Die Erfassung der Signaturen hat aus energetischen Gründen mit einer bestimmten Bandpaßbreite des Meßsensors zu erfolgen. In Erfüllung dieser Anforderungen werden die einzelnen Ausprägungen der spektralen Signatur nicht mit der erforderlichen Präzision abgebildet. Zudem dringt die Strahlung in Abhängigkeit von der Wellenlänge und der Polarisationsausprägung unterschiedlich tief in das Medium (die Haut) ein. Die Folge dieser beiden systematisch wirkenden Effekte ist eine Unsignifikanz und Mehrdeutigkeit des Meßsignals.

Eine Konstanz der Eindringtiefe kann annähernd durch Variation und Anpassung der Strahlungsenergie in Abhängigkeit von der Frequenz und der Polarisation der Meßstrahlung erreicht werden. Demgegenüber erfordert die Rekonstruktion spektraler Feinstrukturen die Anwendung aufwendiger mathematischer und numerischer Korrekturverfahren.

In ihrer Erscheinung stellen die durch Messung gewonnenen Signaturen eine unscharfe Abbildung der "wirklichen" Signaturen dar. Die Unschärfe wird neben den instrumentellen Abbildungsfehlern auch verursacht durch Anlagerungseffekte zwischen den einzelnen Molekülen, durch Dopplereffekte und durch Kollissionseffekte.

Für Flüssigkeiten sind diese Wechselwirkungen zwischen benachbarten Molekülen komplizierter zu beschreiben und ausgeprägter wirkend als in Gasen.

Zielsetzung der Korrekturmaßnahmen ist die Verbesserung des spektralen Auflösungsvermögens, worin auch die Korrektur der durch Moleküldynamik verursachten Bewegungsunschärfe eingeschlossen ist.

Hierzu werden Methoden aus der Kategorie der Rückfaltungs-operationen angewandt. Das Problem besteht dabei in der Erkennbarkeit und der Bewertung der unterschiedlichen Einflußgrößen, die zu einer Verbreiterung und damit zur Unschärfe der Spektrallinien führen.

Insbesondere ist dabei zu berücksichtigen, daß die Verbreiterungsprofile einer exponentiellen Faltungsoperation folgen. Um die Rückfaltung vorzunehmen, muß die instrumentenbedingte Verbreiterung meßtechnisch gering gehalten werden.

Ein grundlegendes Problem bei der Anwendung der Rückfaltungsoperationen stellt der Einfluß statistischer Störungen auf das Meßsignal dar. Es ist deshalb erforderlich, die stochastisch auftretenden Rauscheinflüsse von den substanzspezifischen Signalausprägungen zu unterscheiden. Dies kann einerseits durch eine vorangehende Filterung des Signals erfolgen, aber auch durch Unterdrückung statistischer Störanteile im Rahmen der Rückfaltungsoperation.

Bei der Modellbildung für die Durchführung der Rückfaltungsoperation ist auch die räumliche und die zeitliche Kohärenz der Strahlungsquelle zu berücksichtigen.

Durch die wellenlängenbedingte unterschiedliche Eindringtiefe der Meßstrahlung in das Meßobjekt werden nicht nur unterschiedliche Volumenmengen, sondern wegen der funktionalen Schichtung des Gewebes auch Volumenbereiche unterschiedlicher Zusammensetzung erfaßt. Dies führt dazu, daß die prozentualen Anteile einzelner Substanzen an der Gesamtmenge unterschiedlich bewertet und (bei fälschlicher Unterstellung jeweils gleicher Eindringtiefe) unrichtige Konzentrationswerte für eine Substanz, z. B. den Blutzucker, geschätzt werden.

Die genannte Fehlerquelle kann durch Ermittlung der Veränderungen der spektralen Signatur bei inkrementaler Veränderung der Eindringtiefe behoben werden. Eine solche Bstimmung der spektralen Beiträge einzelner Schichten ist zur Steigerung der Meßpräzision und vor allem bei der Interpretation der Meßwerte sowie zur Unterscheidung einzelner Substanzen erforderlich, da das Meßsignal integralen Charakter aufweist. Die Integralität ist dadurch gekennzeichnet, daß die spektralen Beträge aus allen Bereichen des durch die Eindringtiefe festgelegten Meßvolumens in nicht unterscheidbarem Maße im Meßsignal vorhanden sind.

Eine analytische Betrachtung der einzelnen Beiträge aus verschiedenen Schichten und die dann mögliche Abschätzung der Dämpfung der Meßstrahlung beim Durchgang durch diese Schichten führt zu einer höheren Präzision bei der Ermittlung des Mengenverhältnisse einer spezifischen Substanz, z. B. des Blutzuckers, und damit zu einer Steigerung der Genauigkeit bei dessen Konzentrationsermittlung.

Als mathematische Verfahren kommen Methoden der Inversions-Analyse zur Anwendung. Hierbei wird auf der Grundlage der Werte der Integralmessung auf die räumlich variierenden Einflüsse aus den Einzelschichten in Abhängigkeit von der Wellenlänge und der Polarisation geschlossen. Das praktische Problem bei der Modellbildung ist hierbei die Wahl der Anzahl und die Wahl der Abmessung der einzelnen Schichten unter Zugrundelegung der ermittelbaren Meßwerte dahingehend, daß der dabei entstehende Fehler einen Minimalwert annimmt. Ein weiteres Problem ist die Stabilität des physikalischen Problems dergestalt, daß es sich stetig verhält gegenüber den äußeren Daten, die gemessen werden können.

### Identifizierung und Diskriminierung

Die Vielzahl der im Blut vorhandenen Substanzen und die teilweise Ähnlichkeit der Spektralsignaturen verlangt zu deren Identifizierung und Unterscheidung eine multispektrale Erfassung der Absorptions- bzw. Reflexionsausprägungen. Die unabhängigen Variablen des multivariaten Datensatzes sind dabei die Wellenlänge und die Polarisation.

Durch Anwendung von Verfahren der multivariaten Statistik läßt sich damit das Vorhandensein bestimmter Substanzen in der Meßflüssigkeit nachweisen. Es handelt sich hierbei vor allem um die Methoden der Clusteranalyse, der Diskriminanzanalyse sowie der Varianzanalyse.

Für die Bestimmung der Beiträge einzelner Signaturbeiträge am integralen Meßsignal sind charakteristische isolierte Signaturausprägungen von Einzelsubstanzen aufzufinden und zu quantifizieren. Dies erfolgt durch Bestimmung von Spektralausprägungen spezifischer Substanzen in jenen Wellenlängenbereichen, die mit großer Wahrscheinlichkeit nicht durch Beiträge anderer Substanzen überlagert sind. Im allgemeinen erstreckt sich dieser charakteristische Merkmalsbereich über einen sehr kleinen Wellenlängenumfang; er erlaubt jedoch bereits eine grundsätzlich Bestimmung der Konzentration dieser Substanz aus einem Substanzgemisch.

Im Folgeschritt werden die Beiträge der bezüglich der Absolutmenge identifizierbaren Einzelanteile von der integralen Spektralsignatur abgetrennt. Für weitere Substanzen wird in analoger Weise so lange verfahren, bis die zu identifizierende und quantitativ zu bestimmende Substanz bezüglich ihrer Spekralsignatur isoliert vorliegt.

Die Ermittlung der charakteristischen Clustereigenschaften der zu bestimmenden Substanz wird durch Bestimmung der spektralen Signatur einer Musterprobe definierter Konzentration der Substanz erreicht.

Die für diesen Diskriminierungs- und Isolierungsvorgang angewandten Verfahren sind die Regressions-, die Korrelations- und die Faktorenanalyse. Für die Bestimmung der Beiträge der Einzelsubstanzen hat sich auch die Korrelations- bzw. die Regressionsbeziehung zwischen einzelnen Spektrallinien aus Teilbereichen der Substanzsignatur bewährt.

### E. Beschreibung des neuartigen Systems

Das der Erfindungsidee zugrundeliegende Meßgerät ist einfach aufgebaut und zu bedienen. Es eignet sich daher zur Eigenbestimmung der Konzentration von Blutinhaltsstoffen, insbesondere der Blutzucker- und Alkoholwerte, durch den Probanden selbst, nichtinvasiv, d. h. ohne notwendige Blutentnahme, direkt durch die Haut hindurch.

Zur Vornahme des Meßvorgangs wird eine ebene Auflagefläche, die am Ende einer dünnen Meßleitung angebracht ist, für einige Sekunden an eine genügend transparente Stelle des menschlichen Körpers schlüssig angedrückt. Als geeignet haben sich hierbei das Ohrläppchen, die Unterlippe und die Zungenspitze erwiesen.

Der gesamte Meßvorgang erfolgt physikalisch ohne Zuhilfenahme chemischer Substanzen; die Meßwerterfassung, - aufbereitung, -auswertung und -speicherung wird ausschließlich in digitaler Form vorgenommen.

Die konsequente Digitalisierung des Meßverfahrens erleichtert den Meß- und den Eichvorgang erheblich. Letzterer wird unter Zuhilfenahme digitalisierter Referenzspektren sowohl für die Strahlungsquelle als auch die Referenzsubstanzen und die wesentlichen Blutinhaltsstoffe geräteintern vorgenommen und erfordert keine externe Einflußnahme. Zudem erlaubt diese Vorgehensweise auch die Erfassung und Korrektur von physikalisch und technisch bedingten Strahlungsverfälschungen.

Eine kohärente oder inkohärente Strahlungsquelle emittiert Infrarotstrahlung einer bestimmten Energiedichte über einen bestimmten Wellenlängenbereich. Der Umfang des Wellenlängenbereichs wird nach den Kriterien der Meßaufgabe festgelgt. Bei einem Universalgerät wird ein größerer Wellenlängenbereich (z. B. von 0,8 µm bis 30 µm) gefordert, bei Spezialausführungen (z. B. zur Blutzucker- oder Alkoholbestimmung) ist ein entsprechend kleinerer Wellenlängenumfang ausreichend. Bei Verwendung einer kohärenten Strahlungsquelle (Laser) wird für den Allgemeinfall eine Durchstimmbarkeit und eine Aneinanderfügung von Strahlungsquellen unterschiedlicher Wellenlängenbereiche notwendig. Inkohärente Strahlungsquellen weisen demgegenüber bereits eine große Emissionsbandbreite auf.

Die Energieversorgung kann entweder intern (durch Batterien) oder extern (z. B. durch Anschluß an das Netz oder einen Zigarettenanzüder im Kraftfahrzeug) erfolgen. Für eine am Körper des Probanden tragbare Ausführung können Batterien in einer Gürteltasche mitgeführt werden.

Für den Meßvorgang ist die von der Strahlungsquelle emittierte Strahlung zu modulieren. Diese Modulation kann in drei Stufen erfolgen, wobei die Stufenfolge variiert werden kann.

In einer Stufe wird die Bandbreite der Meßstrahlung festgelegt. Dieser Vorgang erfolgt durch Einschaltung eines Bandpaßfilters, das Strahlung eines eng begrenzten Wellenlängenbereichs passieren läßt.

Zur Durchführung der Messung ist es erforderlich, die Zentralfrequenz des Bandpaßfilters bei konstanter Paßbandbreite über einen großen Wellenlängenbereich zu verschieben. Dieser Effekt kann duch Kombination mehrerer Einzelfilter erzielt werden. Wesentlich ist hierbei eine Abstimmung der Filtercharakteristik im Hinblick auf die verfügbare Energie der Meßstrahlung und im Hinblick auf die zu erzielende Eindringtiefe der Strahlung in das Meßobjekt.

Bei Verwendung kohärenter durchstimmbarer Strahlungsquellen kann eine beschränkte Durchstimmbarkeit der Strahlungsquelle erreicht werden. Eine Bandbreitenbegrenzung der Meßstrahlung wird jedoch auch in diesem Fall notwendig und von Vorteil sein. Auch hier muß eine Abstimmung der Strahlungsenergie bezüglich der Bandbreite und der Wellenlänge vorgenommen werden.

In einer weiteren Stufe erfolgt eine gerichtete Polarisierung der Meßstrahlung (wobei grundsätzlich die Emission zufallspolarisierter Strahlung durch die Strahlungsquelle ausgegangen wird). Die bevorzugten Polarisationsmoden sind die (originäre) Zufallspolarisation, die Horizontal- (H) und Vertikalpolarisation (V), Links- und Rechtsdrehung der Polarisationsrichtung der Meßstrahlung.

Damit ist die Polarisationsausprägung der Meßstrahlung vor Wechselwirkung mit dem Meßobjekt festgelegt.

Nach Wechselwirkung der Meßstrahlung mit dem Meßobjekt wird der Anteil einer bevorzugten Polarisationsrichtung detektiert. Dabei werden die gleichen Polarisationsausprägungen gewählt wie für den Meßstrahl vor Wechselwirkung mit dem Meßobjekt. Auf diese Weise läßt sich eine Kombination verschiedener Polarisationmoden der Strahlung erreichen, z. B. VV, VH, HV HH, usw..

Durch diese Vorgehensweise wird die Anzahl der durch die Strahlungsfrequenz gegebenen Meßvariablen um den Satz der durch die Polarisationsmodi gegebenen Meßvariablen dergestalt erhöht, daß neben den Multispektralsignaturen auch die Multipolarisationssignaturen erfaßt werden als Erkennung und Unterscheidung verschiedener Substanzen.

In einer weiteren Stufe wird der Fluß der Meßstrahlung in zeitlicher Folge unterbrochen. Die damit erzielte Pulsierung kann spontan oder transient (z. B. sinusförmig) erfolgen. Der Zweck dieser Strahlungsmanipulation ist eine Beeinflussung der Eindringtiefe. Die vorliegenden Erfahrungswerte für die Eindringtiefe basieren auf einer kontinuierlichen Strahlungsbeaufschlagung des Meßobjekts. Durch eine kurzzeitige Strahlungsbeaufschlagung wird eine Erhöhung der Eindringtiefe erzielt. Die Dauer dieser Kurzzeitigkeit ist von den optischen Eigenschaften des Meßobjekts, der Wellenlänge und der Polarisation abhängig und muß für die Bestimmung spezifischer Meßsubstanzen individuell festgelegt werden. Für eine spezifische Substanz kann sie bei verschiedenen Probanden als gleichbleibend angesehen werden.

Die Zuleitung der modulierten Strahlung zum ATR-Element erfolgt in einem optisch abgeschirmten Lichtwellenleiter, um störende Einflüsse durch Streulicht zu vermeiden. (Analog wird auch bei der Ableitung der Meßstrahlung vom ATR-Element zum Sensorelement verfahren).

Die Einkopplung der Strahlung in das ATR-Element erfolgt entweder unter einer konstanten oder einem variablen Winkel. Die Art der Einkopplung bestimmt in gegenseitiger Abhängigkeit auch die Art der Ausführung des ATR-Elements.

Bei einer prismenförmigen Ausführung des ATR-Elements sind dem Einkopplungswinkel zur Gewährleistung der Funktionsfähigkeit enge Grenzen gesetzt. Eine Veränderung des Winkels innerhalb der zulässigen Grenzen führt zu einer Verlagerung der Meßpunkte am Meßobjekt. Dies kann Mehrdeutigkeit der Meßsignals zur Folge haben. Dieser Effekt wird durch die Verwendung einer halbkugelförmigen Oberfläche des ATR-Elements vermieden. Zur Erhöhung der Wechselwirkung der Strahlung mit dem Meßobjekt wird das ATR-Element an der Auflagefläche zum Objekt noch mit verschiedenen dünnen Oberflächenschichten versehen, die als Distanz- und Resonanzschichten wirken.

Im einzelnen Fall ist die konstruktive Gestaltung des ATR-Elements von der Meßaufgabe abhängig.

Nach Passieren des zweiten Polarisationsfilters gelangt die Meßstrahlung zum Strahlungssensor. Nach Digitalisierung des analogen Intensitätssignals der wellenlängen- und polaristionsmodus-abhängigen Strahlungsintensität erfolgt die Auswertung und die Speicherung des Konzentrationswertes des Inhaltsstoffes.

Im wesentlichen wird mit Hilfe der Auswertung die Spektralsignatur gesuchter Substanzen identifiziert, von den Spektalsignaturen anderer Texturen diskriminiert und als Ereignis einer Zeitserie gespeichert.

Die gewonnenen Meßwerte können digital auf Datenträgern abgespeichert und dann in Losen unterschiedlicher Größe ausgewertet werden. Damit lassen sich Hinweise auf probandenspezifische Verhalten ableiten, das z. B. zur Auslösung einer Warnfunktion oder als Aufforderung für die Verabreichung eines bestimmten Medikaments ausgelegt werden kann.

Darüberhinaus kann man die Meßwerte problemlos auf Computer in Arztpraxen und Kliniken zur weiteren Verwendung übertragen.

Bezüglich der Größe des Gerätes dürften zwei Ausführungen von marktwirtschaftlicher Bedeutung sein, nämlich ein solches in der Größe eines tragbaren PC für die Arztpraxis und ein solches von der Größe eines Walkman, das vom Probanden am Körper getragen werden kann.

**Tabelle 1:**

| *Typische Wellenzahlen zur spektroskopischen Identifizierung einiger biologisch relevanter Substanzen* | | |
|---|---|---|
| *Substanz* | *Anregung* | *Wellenzahl (cm*^{*-1*}*)* |
| Wasser | sym Streckung | 3652 |
| | assym Streckung | 3756 |
| | Scherung | 1586 |
| Alkohole | OH-Streckung | 3600 - 3200 |
| | OH-Knickung | 11500 - 1300 |
| | CO-Streckung | 19220 - 1000 |
| | | |
| Aromate | CH-Streckung | 3300 |
| | CC-Streckung | 11600 |
| | | |
| Amine | NH-Streckung | 3500 - 31300 |
| | | |
| Carboxyl-Säure | CO-Streckung | 1740 - 120 |
| | | |
| Amide | CO-Streckung | 1670 - 1640 |
| | NH-Streckung | 3500 - 3100 |
| | NH-Knickung | 1640 - 1550 |
| | | |
| Lipide | CH-Streckung | 2300 - 2350 |
| | CH2,CH3-Strckg | 1680 - 1760 |
| | | |
| Proteine | | 2080 - 2220 |
| | NH-deformation | 1560 - 1670 |
| | | |
| Carbohydrate | CO, OH | 2060 - 2150 |

## Patentansprüche

1. Meßgerät zur Ermittlung des Konzentrationsgehaltes von in Flüssigkeit, zum Beispiel Blut, enthaltenen und/oder gelösten Substanzen, bestehend aus mindestens einer Strahlungsquelle zur Erzeugung von Strahlung, einem Strahlungs- oder Meßsensor, wobei der Sensor in einer als ATR-Element (attenuated total reflection) ausgebildeten optischen Vorrichtung, angeordnet ist, **dadurch gekennzeichnet, daß** die als ATR-Element ausgebildete optische Vorrichtung als Schichtung optischer Materialien mit verschiedenen Brechungskoeffizienten gebildet ist und daß die Schichten mit verschiedenen Brechungskoeffizienten in einem bestimmten Abstand zueinander angeordnet sind.

2. Meßgerät nach Anspruch 1, **dadurch gekennzeichnet, daß** das ATR-Element mit verschieden dünnen Oberflächenschichten versehen ist.

3. Meßgerät nach einem oder beiden der vorstehenden Ansprüche, **dadurch gekennzeichnet, daß** das ATR-Element eine halbkugelförmige Ausbildung besitzt.

4. Meßgerät nach einem oder mehreren der vorstehenden Ansprüche, **dadurch gekennzeichnet, daß** das Material der Halbkugel und das der äußeren Resonanzschicht einen hohen Brechungsindex n₁ bzw. n₃ und das der dazwischenliegenden Abstandsschicht einen niedrigeren Brechnungsindex n₂ aufweist, wobei n₁ = n₃ und damit n₂ < n₁ bzw. n₂ < n₃ wird.

5. Meßgerät nach einem oder mehreren der vorstehenden Ansprüche **dadurch gekennzeichnet, daß** die Strahlungsquelle zur Erzeugung von Strahlung als kohärente Strahlungsquelle, beispielsweise eine Laserstrahlungsquelle, ausgebildet ist.

6. Meßgerät nach einem oder mehreren der vorstehenden Ansprüche **dadurch gekennzeichnet, daß** die Strahlungsquelle als inkohärente Strahlungsquelle, beispielsweise als Nernst-Strahler ausgebildet ist.

7. Meßgerät nach einem oder mehreren der vorstehenden Ansprüche, **dadurch gekennzeichnet, daß** an der Strahlungsquelle eine Einrichtung zur Veränderung beziehungsweise Begrenzung der Strahlungsfrequenz angeordnet ist.

8. Meßgerät nach einem oder mehreren der vorstehenden Ansprüche, **dadurch gekennzeichnet, daß** die Einrichtung zur Veränderung beziehungsweise Begrenzung der Strahlungsfrequenz ein Bandpaßfilter ist.

9. Meßgerät nach einem oder mehreren der vorstehenden Ansprüche, **dadurch gekennzeichnet, daß** eine Einrichtung zur Gewährleistung einer konstanten frequenzabhängigen Energiedichte an der Strahlungsquelle angeordnet beziehungsweise dieser zugeordnet ist.

10. Meßgerät nach einem oder mehreren der vorstehenden Ansprüche, **dadurch gekennzeichnet, daß** in dem Gerät mindestens eine Einrichtung zur Strahlungsmodulation angeordnet ist.

11. Meßgerät nach einem oder mehreren der vorstehenden Ansprüche, **dadurch gekennzeichnet, daß** eine kohärente Strahlungsquelle vorhanden ist, deren Frequenzen durchstimmbar sind und/oder daß Strahlungsquellen unterschiedlicher Wellenlängenbereiche vorhanden sind.

12. Meßgerät nach einem oder mehreren der vorstehenden Ansprüche, **dadurch gekennzeichnet, daß** inkohärente Strahlungsquelle vorhanden ist, die eine große Emmissionsbandbreite aufweist.

13. Meßgerät nach einem oder mehreren der vorstehenden Ansprüche, **dadurch gekennzeichnet, daß** eine Strahlführung Bestandteil des Gerätes ist, die derart ausgebildet ist, daß sie eine genügend tiefe Eindringung des Meßstrahls bei maximal häufiger Wechselwirkung der Meßsubstanz und Totalreflektion gewährleistet.

14. Meßgerät nach einem oder mehreren der vorstehenden Ansprüche, **dadurch gekennzeichnet, daß** die Strahlführung eine schlüssige beziehungsweise ebene Auflagefläche am Meßort aufweist.

15. Meßgerät nach einem oder mehreren der vorstehenden Ansprüche, **dadurch gekennzeichnet, daß** die schlüssige beziehungsweise ebene Auflagefläche am Ende einer dünnen Meßleitung angeordnet ist.

16. Meßgerät nach einem oder mehreren der vorstehenden Ansprüche, **dadurch gekennzeichnet, daß** zwischen Laser und ATR-Element sowie zwischen ATR-Element und Strahlungsund Meßsensor Lichtwellenleiter angeordnet sind.

17. Meßgerät nach einem oder mehreren der vorstehenden Ansprüche, **dadurch gekennzeichnet, daß** zwischen Laser und ATR-Element sowie zwischen ATR-Element und Strahlungsund Meßsensor jeweils ein Lichtwellenleiter angeordnet ist, durch den sowohl die Meßstrahlung als auch die empfangene Strahlung geleitet wird.

18. Meßgerät nach einem oder mehreren der vorstehenden Ansprüche, **dadurch gekennzeichnet, daß** der Lichtwellenleiter aus einer kristallinen Faser besteht und hyperbolisch geformte Faserenden zur Verzweigung des Strahlweges aufweist.

19. Meßgerät nach einem oder mehreren der vorstehenden Ansprüche, **gekennzeichnet durch** eine Einrichtung zur Meßwerterfassung, Auswertung und Speicherung.

20. Meßgerät nach einem oder mehreren der vorstehenden Ansprüche, **dadurch gekennzeichnet, daß** in der Auswerteeinrichtung ein Speicher für zu hinterlegende Werte angeordnet ist.

21. Meßgerät nach einem oder mehreren der vorstehenden Ansprüche, **gekennzeichnet durch** eine Einrichtung zur Digitalisierung.

22. Meßgerät nach einem oder mehreren der vorstehenden Ansprüche, **dadurch gekennzeichnet, daß** zwischen Lichtwellenleiter und ATR-Element beziehungsweise zwischen Lichtwellenleiter und Strahlungs- und Meßsensor eine Kombination mehrerer Einzelfilter angeordnet ist.

23. Meßgerät nach einem oder mehreren der vorstehenden Ansprüche, **dadurch gekennzeichnet, daß** ein Detektor derart angeordnet ist, daß er nach Wechselwirkung der Meßstrahlung mit dem Meßobjekt den Anteil einer bevorzugten Polarisationsrichtung detektiert.

24. Meßgerät nach einem oder mehreren der vorstehenden Ansprüche, **dadurch gekennzeichnet, daß** Koppeleinrichtungen zur Einkoppelung der austretenden und eintreffenden Strahlen sowohl an dem ATR-Element als auch an dem Strahlungs- und Meßsensor angeordnet sind.

25. Meßgerät nach einem oder mehreren der vorstehenden Ansprüche, **dadurch gekennzeichnet, daß** eine Einrichtung zur Pulsierung der Meßstrahlung vorhanden ist.

26. Verfahren zur Untersuchung des Konzentrationsgehalts von in Flüssigkeit, zum Beispiel Blut, enthaltenen und/oder gelösten Substanzen, bei dem eine Meßstrahlung von einer Meßeinrichtung in die zu messende Flüssigkeit in vivo, ex vivo oder in vitro eingeleitet, die von der Flüssigkeit reflektierte Strahlung mittels einer Empfangseinrichtung aufgefangen und einer Auswerteeinrichtung zugeführt wird, **dadurch gekennzeichnet, daß** ein Meßgerät nach einem oder mehreren der vorhergehenden Patentansprüche 1 bis 25 verwendet wird.

27. Verfahren nach dem vorhergehenden Anspruch 26, **dadurch gekennzeichnet, daß** die empfangenen Daten mit in der Empfangs- und Auswerteeinrichtung hinterlegten Daten derart verglichen werden, daß mittels eines in der Auswerteeinrichtung hinterlegten Modells auf der Grundlage von Werten für die räumlich variablen Einflüsse aus den Einzelschichten des Gewebemodells in Abhängigkeit von der Wellenlänge und der Polarisation geschlossen wird und damit das Vorhandensein bestimmter Substanzen in der Meßflüssigkeit nachgewiesen wird.

28. Verfahren nach einem oder beiden der vorhergehenden Ansprüche 26 und 27, **dadurch gekennzeichnet, daß** der Nachweis des Vorhandenseins bestimmter Substanzen mittels solcher Methoden wie Clusteranalyse, Diskriminanzanalyse oder Varianzanalyse erfolgt.

29. Verfahren nach einem oder mehreren der vorhergehenden Ansprüche 26 bis 28, **dadurch gekennzeichnet, daß** das Verfahren beliebig häufig jederzeit wiederholbar durchführbar ist.

30. Verfahren nach einem oder mehreren der vorhergehenden Ansprüche 26 bis 29, **dadurch gekennzeichnet, daß** sowohl die ausgesandte Meßstrahlung als auch die empfangene Strahlung durch mindestens ein ATR-Element und über mindestens ein optisches Element unter Ausnutzung substanzspezifischer Absorptions- beziehungsweise Reflektionseigenschaften geführt wird.

31. Verfahren nach einem oder mehreren der vorhergehenden Ansprüche 26 bis 30, **dadurch gekennzeichnet, daß** die Meßstrahlung derart ausgebildet ist, daß sie eine die Flüssigkeit umschließende Begrenzungsschicht, wie zum Beispiel eine Trennwand durchdringt, bevor sie in die Flüssigkeit eintritt und die reflektierte Strahlung durch die Begrenzungsschicht danach zum Meßsensor gelangt.

32. Verfahren nach einem oder mehreren der vorhergehenden Ansprüche 26 bis 31, **dadurch gekennzeichnet, daß** der Meßsensor, die Begrenzungsschicht beziehungsweise Trennwand und der Empfangssensor zur Verbesserung der Ein- und Auskopplung der Meßstrahlung am Kontaktort der Meßvorrichtung mit dem Meßobjekt mit einer dünnen Schicht einer festen beziehungsweise pulverigen oder flüssigen, aktiven Substanz bestrichen werden.

33. Verfahren nach einem oder mehreren der vorstehenden Ansprüche 26 bis 32, **dadurch gekennzeichnet, daß** die Meßstrahlung mittels einer optischen Vorrichtung in einen optischen Resonanzzustand versetzt wird.

34. Verfahren nach einem oder mehreren der vorstehenden Ansprüche 26 bis 33, **dadurch gekennzeichnet, daß** die Strahlung einer einheitlichen Polarisationsausprägung verwendet wird.

35. Verfahren nach einem oder mehreren der vorstehenden Ansprüche 26 bis 34, **dadurch gekennzeichnet, daß** die Polarisationsebene im Verlauf der Messung über den Gesamtbereich aller Polarisationsebenen geändert wird, wobei jedoch die Horizontal- und die Vertikalpolarisation besondere Beachtung findet.

36. Verfahren nach einem oder mehreren der vorstehenden Ansprüche 26 bis 35, **dadurch gekennzeichnet, daß** bei der Änderung der Polarisationsebenen zusätzlich dynamische Variationen vorgenommen werden, wie zum Beispiel Zirkular- und eliptische Polarisation und links- und rechtsdrehende Polarisation.

37. Verfahren nach einem oder mehreren der vorstehenden Ansprüche 26 bis 36, **dadurch gekennzeichnet, daß** das Meßobjekt mit einer diskontinuierlichen Strahlungsbeaufschlagung, wie zum Beispiel einer Pulsierung, beaufschlagt wird.

38. Verfahren nach einem oder mehreren der vorstehenden Ansprüche 26 bis 37, **dadurch gekennzeichnet, daß** als Meßstrahlung Infrarotstrahlung in einem Gesamtbereich von 0,8 µm bis 100 µm, im nahen Infrarotbereich von 0,8 µm bis 2,5 µm, im mittleren Infrarotbereich von 2,5 µm bis 15 µm und im fernen Infrarotbereich von 15 µm bis 100 µm verwendet wird.

39. Verfahren nach einem oder mehreren der vorstehenden Ansprüche 26 bis 38, **dadurch gekennzeichnet, daß** eine Variation und Anpassung der Strahlungsenergie in Abhängigkeit von Frequenz und Polarisation der Meßstrahlung erfolgt.

40. Verfahren nach einem oder mehreren der vorstehenden Ansprüche 26 bis 39, **dadurch gekennzeichnet, daß** zur Feststellung der Absorptionskoeffizienten für alle drei räumlichen Richtungen einer anisotropen Meßprobe eine Kombination des Reflektionsgrades für verschiedene Polarisationsebenen der Meßstrahlung erfolgt.

41. Verfahren nach einem oder mehreren der vorstehenden Ansprüche 26 bis 40, **gekennzeichnet durch** die Anwendung (Verwendung) der Methode der Rückfaltungs-Operation.

42. Verfahren nach einem oder mehreren der vorstehenden Ansprüche 26 bis 41, **dadurch gekennzeichnet, daß** zur Unterscheidung von substanzspezifischen Signalausprägungen eine vorangehende Filterung des bzw. der Signale erfolgt.

43. Verfahren nach einem oder mehreren der vorstehenden Ansprüche 26 bis 42, **dadurch gekennzeichnet, daß** bei der Modellbildung die räumliche und die zeitliche Kohärenz der Strahlungsquelle Berücksichtigung findet.

44. Verfahren nach einem oder mehreren der vorstehenden Ansprüche 26 bis 43, **gekennzeichnet durch** die Anwendung (Verwendung) der Inversions-Analyse.

45. Verfahren nach einem oder mehreren der vorstehenden Ansprüche 26 bis 44, **dadurch gekennzeichnet, daß** die Meßstrahlen von einer kohärenten oder inkohärenten Strahlungsquelle zur Verfügung gestellt werden.

46. Verfahren nach einem oder mehreren der vorstehenden Ansprüche 26 bis 45, **dadurch gekennzeichnet, daß** ein Selektions- bzw. Verstärkungseffekt des Meßsignales derart erreicht wird, daß eine reaktive Substanz in die Flüssigkeit vor der Messung eingebracht wird und diese Substanz durch die Meßstrahlung oder einer anderen Strahlungsart zu einer Reaktion angeregt wird, wobei diese Reaktion zu einer vorübergehenden oder bleibenden Änderung der Signatur der von der Flüssigkeit und/oder deren Inhaltssubstanzen emittierten Strahlungen führen.

47. Verfahren nach einem oder mehreren der vorstehenden Ansprüche 26 bis 46, **dadurch gekennzeichnet, daß** zum Nachweis extrem niederer Konzentrationen in Flüssigkeiten die provozierte Reaktion mittels eines spezifisch eingebrachten Reaktionsstoffes derart angeregt wird, daß sie über dem bzw. den stochastischen oder systematischen Störpegeln liegende Signalausprägungen erzeugen.

48. Verfahren nach einem oder mehreren der vorstehenden Ansprüche 26 bis 47, **dadurch gekennzeichnet, daß** die dem Sensor zugewandte Außenseite der Trennwand oder die Sensoraußenfläche mit einer aktiven Substanz bestrichen werden, die zu einer selektiven Minderung bzw. Verstärkung und damit zu einer signifikanten Ausprägung der zum Nachweis einer Substanz bzw. einer Reaktion erforderlichen charakteristischen Strahlungssignatur führt.

49. Verfahren nach einem oder mehreren der vorstehenden Ansprüche 26 bis 48, **dadurch gekennzeichnet, daß** der Eingang der Meßstrahlung in das und der Ausgang der Meßstrahlung aus dem Meßobjekt an der gleichen Stelle am Meßobjekt erfolgt.

50. Verfahren nach einem oder mehreren der vorstehenden Ansprüche 26 bis 49, **dadurch gekennzeichnet, daß** aus den verfügbaren Wellenlängenbereichen der Meßstrahlung jeweils nur ein definiertes Frequenzband zur Beaufschlagung des Meßobjektes herangezogen wird und dieses Frequenzband über den zur Bestimmung spezifischer Inhaltsstoffe erforderlichen Frequenzbereich kontinuierlich verschoben wird und daß dieser Vorgang während der Zeit der Messung einmalig oder mehrfach erfolgt, wobei je nach Wellenlänge der Strahlung auch deren Energie verändert wird.

51. Verfahren nach einem oder mehreren der vorstehenden Ansprüche 26 bis 50, **dadurch gekennzeichnet, daß** die Manipulation der Meßstrahlung nicht nur im Strahlengang zwischen der Strahlenquelle und dem Meßobjekt vor der Strahlungsbeaufschlagung des Meßobjektes, sondern auch im Strahlengang zwischen Meßobjekt und Meßsensor nach erfolgter Strahlungsbeaufschlagung des Meßobjektes vorgenommen wird.

52. Verfahren nach einem oder mehreren der vorstehenden Ansprüche 26 bis 51, **dadurch gekennzeichnet, daß** die Meßstrahlung in die zur Kontaktherstellung mit dem Meßobjekt verwendeten optischen Elemente unter einem veränderlichen spezifischen Winkel zur Erreichung unterschiedlicher Wechselwirkungen der Strahlung mit dem Meßobjekt eingekoppelt wird.

53. Verfahren nach einem oder mehreren der vorstehenden Ansprüche 26 bis 52, **dadurch gekennzeichnet, daß** die vom Meßobjekt zurückkehrende Strahlung im selben oder in einem anderen Strahlenleitungselement als die das Meßobjekt beaufschlagende Meßstrahlung zu einem Strahlungssensor geleitet wird.

54. Verfahren nach einem oder mehreren der vorstehenden Ansprüche 26 bis 53, **dadurch gekennzeichnet, daß** das erfaßte Meßsignal der vom Meßobjekt reflektierten Strahlung in digitale Form überführt und dann durch geeignete Maßnahmen von systematischen und statistischen Meßfehlern befreit beziehungsweise deren Einfluß vermindert wird.

55. Verfahren nach einem oder mehreren der vorstehenden Ansprüche 26 bis 54, **dadurch gekennzeichnet, daß** das Meßsignal aus dem zu bestimmenden Inhaltsstoff vom Meßsignal anderer Inhaltsstoffe abgetrennt und die Natur des zu bestimmenden Inhaltsstoffes und damit sein Vorhandensein eindeutig nachgewiesen wird.

## Claims

1. Measuring instrument for determining the concentration of substances contained and/or solved in a liquid, for example blood, comprising at least one radiation source for producing radiation, a radiation or measuring sensor, the sensor being arranged in an optical device, which is designed as an ATR element (attenuated total reflection), **characterised in that** the optical device, designed as ATR element, is arranged in layers of optical materials with different refractive indices, and that the layers with different refractive indices are arranged in a defined distance to each other.

2. Measuring instrument according to claim 1, **characterised in that** the ATR element has differently thin surface layers.

3. Measuring instrument according to one or both of the preceding claims, **characterised in that** the ATR element has a hemispherical design.

4. Measuring instrument according to one or more of the preceding claims, **characterised in that** the material of the hemisphere and the material of the outer resonance layer have a high refractive index n1, respectively n3, and that the material of the intermediate distance layer has a lower refractive index n2, in which n1 = n3, and thus n2 < n1, respectively n2 < n3.

5. Measuring instrument according to one or more of the preceding claims, **characterised in that** the radiation source for producing radiation is designed as a coherent radiation source, for example as a laser beam source.

6. Measuring instrument according to one or more of the preceding claims, **characterised in that** the radiation source is designed as a incoherent radiation source, for example as a Nernst radiator.

7. Measuring instrument according to one or more of the preceding claims, **characterised in that** at the radiation source an arrangement for altering, respectively limiting, the radiation frequency is provided.

8. Measuring instrument according to one or more of the preceding claims, **characterised in that** the arrangement for altering, respectively limiting, the radiation frequency is a band-pass filter.

9. Measuring instrument according to one or more of the preceding claims, **characterised in that** an arrangement for securing a constant frequency-dependent energy density is arranged at the radiation source, respectively assigned to it.

10. Measuring instrument according to one or more of the preceding claims, **characterised in that** in the instrument at least one arrangement for radiation modulation is provided.

11. Measuring instrument according to one or more of the preceding claims, **characterised in that** there is a coherent radiation source, whose frequencies are variable, and/or that there are radiation sources with different wave length ranges.

12. Measuring instrument according to one or more of the preceding claims, **characterised in that** there is an incoherent radiation source, which has a large emission range.

13. Measuring instrument according to one or more of the preceding claims, **characterised in that** a beam control is part of the instrument, the beam control being designed in such a way that it guarantees a sufficiently deep penetration of the measuring beam while the interaction of the sample and the full reflection is maximum.

14. Measuring instrument according to one or more of the preceding claims, **characterised in that** the beam control has a conclusive, respectively even, supporting surface at the measuring point.

15. Measuring instrument according to one or more of the preceding claims, **characterised in that** the conclusive, respectively even, supporting surface is arranged at the end of a thin testing wire.

16. Measuring instrument according to one or more of the preceding claims, **characterised in that** between laser and ATR element as well as between ATR element and radiation and measuring sensor beam waveguides are arranged.

17. Measuring instrument according to one or more of the preceding claims, **characterised in that** between laser and ATR element as well as between ATR element and radiation and measuring sensor each a beam waveguide is arranged through which the measuring radiation as well as the received radiation is guided.

18. Measuring instrument according to one or more of the preceding claims, **characterised in that** the beam waveguide comprises a crystalline fibre and has hyperbolic formed fibre ends for forking the beam path.

19. Measuring instrument according to one or more of the preceding claims, **characterised by** an equipment for collecting, evaluation and storing of measured values.

20. Measuring instrument according to one or more of the preceding claims, **characterised in that** in the evaluation equipment a store for the values to be deposited is arranged.

21. Measuring instrument according to one or more of the preceding claims, **characterised by** an equipment for digitalisation.

22. Measuring instrument according to one or more of the preceding claims, **characterised in that** between beam waveguide and ATR element , respectively between beam waveguide and radiation and measuring sensor a combination of several single filters is arranged.

23. Measuring instrument according to one or more of the preceding claims, **characterised in that** a detector is arranged in such a way that it detects, after interaction of the measuring radiation with the test object, the part of a preferred polarising direction.

24. Measuring instrument according to one or more of the preceding claims, **characterised in that** coupling equipment for coupling the emerging and arriving beams is arranged at the ATR element as well as at the radiation and measuring sensor.

25. Measuring instrument according to one or more of the preceding claims, **characterised in that** there is an arrangement for the pulsation of the measuring radiation.

26. Method for the analysis of the concentration of substances contained and/or dissolved in liquids, for example in blood, where a measuring radiation is introduced from a measuring device into the liquid to be measured in vivo, ex vivo or in vitro, the radiation reflected by the liquid is picked up by means of a receiving appliance and transferred to an evaluation appliance, **characterised in that** a measuring instrument according to one or more of the preceding claims 1 to 25 is used.

27. Method according to the preceding claim 26, **characterised in that** the received dates are compared with the dates deposited in the receiving and evaluation appliance in such a way that by means of a model deposited in the evaluation appliance based on the values for the spatially variable influences out of the individual layers of the tissue model it is concluded depending on the wave length and the polarisation and therefore the presence of certain substances in the test liquid is proved.

28. Method according to one or both of the preceding claims 26 and 27, **characterised in that** the proof of presence of certain substances is carried out by such methods like cluster analysis, discriminance analysis or variance analysis.

29. Method according to one or more of the preceding claims 26 to 28, **characterised in that** the method can be carried out unlimitedly frequently recursively at any time.

30. Method according to one or more of the preceding claims 26 to 29, **characterised in that** the emitted measuring radiation as well as the received radiation is guided through at least one ATR element and over at least one optical element using absorption, respectively reflection, properties specific for the substance.

31. Method according to one or more of the preceding claims 26 to 30, **characterised in that** the measuring radiation is designed in such a way that it penetrates a terminating layer enveloping the liquid, like, for example, a partition wall before it enters the liquid and the reflected radiation reaches then the measuring sensor through the terminating layer.

32. Method according to one or more of the preceding claims 26 to 31, **characterised in that** the measuring sensor, the terminating layer, respectively the partition wall and the receiving sensor are covered with a thin layer of a solid, respectively powder or liquid, active substance in order to improve the connection and disconnection of the measuring radiation with the test object at the contact point of the measuring device.

33. Method according to one or more of the preceding claims 26 to 32, **characterised in that** the measuring radiation is brought into an optical resonance state by means of an optical device.

34. Method according to one or more of the preceding claims 26 to 33, **characterised in that** the radiation of a uniform polarisation characteristic is used.

35. Method according to one or more of the preceding claims 26 to 34, **characterised in that** the plane of polarisation changes during the measuring across the total field of all planes of polarisation, however, with special regard to the horizontal and vertical polarisation.

36. Method according to one or more of the preceding claims 26 to 35, **characterised in that** during the change of the planes of polarisation additionally dynamic variations are carried out, for example circular and elliptic polarisation and left-and right-handed polarisation.

37. Method according to one or more of the preceding claims 26 to 36, **characterised in that** the test object is impinged on a discontinuous radiation admission, for example a pulsation.

38. Method according to one or more of the preceding claims 26 to 37, **characterised in that** infrared radiation is used as a measuring radiation in a total range of 0.8 to 100 micrometers, in the close infrared range of 0.8 to 2.5 micrometers, in the middle infrared range of 2.5 to 15 micrometers and in the distant infrared range of 15 to 100 micrometers.

39. Method according to one or more of the preceding claims 26 to 38, **characterised in that** variation and adaptation of the radiation energy depending on the frequency and polarisation of the measuring radiation is done.

40. Method according to one or more of the preceding claims 26 to 39, **characterised in that** for the identification of the absorption coefficient for all three spatial directions of an anisotropic test sample a combination of the degree of reflection of different planes of polarisation of the measuring radiation is carried out.

41. Method according to one or more of the preceding claims 26 to 40, **characterised by** the application (use) of the method of the backfolding operation.

42. Method according to one or more of the preceding claims 26 to 41, **characterised in that** in order to distinguish signal characteristics specific for a substance first a filtration of the signal, respectively the signals, is carried out.

43. Method according to one or more of the preceding claims 26 to 42, **characterised in that** for the designing of a model the space and time coherency of the radiation source is taken into consideration.

44. Method according to one or more of the preceding claims 26 to 43, **characterised by** the application (use) of the inversion analysis.

45. Method according to one or more of the preceding claims 26 to 44, **characterised in that** the measuring beams are provided by a coherent or incoherent radiation source.

46. Method according to one or more of the preceding claims 26 to 45, **characterised in that** a selection, respectively intensification, effect of the measuring signal is reached in such a way that a reactive substance is put into the liquid before measuring, and this substance is stimulated by means of the measuring radiation or another kind of radiation to a reaction, this reaction leading to a temporary or permanent change of the signature of the radiation emitted by the liquid and/or its contents.

47. Method according to one or more of the preceding claims 26 to 46, **characterised in that** for the proof of extremely low concentrations in liquids the provoked reaction is stimulated by means of a specifically introduced reaction substance in such a way that they produce signal characteristics lying above the stochastic or systematic interference level, respectively levels.

48. Method according to one or more of the preceding claims 26 to 47, **characterised in that** the outer surface of the partition wall facing the sensor or the outer surface of the sensor are covered with an active substance, which leads to a selective reduction, respectively intensification, and thus leads to a significant characteristic of the characteristic radiation signature necessary for the proof of a substance, respectively a reaction.

49. Method according to one or more of the preceding claims 26 to 48, **characterised in that** the measuring radiation enters and leaves the test object at the same point of the test object.

50. Method according to one or more of the preceding claims 26 to 49, **characterised in that** out of the available wave length ranges of the measuring radiation only one defined frequency band each is used to impinge on the test object, and this frequency band is shifted continuously across the frequency range necessary for the determination of specific contents, and that this procedure is carried out during the measuring period once or several times, their energy also being changed depending on the wave length of the radiation.

51. Method according to one or more of the preceding claims 26 to 50, **characterised in that** the manipulation of the measuring radiation is done not only in the beam path between the radiation source and the test object before the test object is impinged with radiation, but also in the beam path between the test object and the measuring sensor after the test object has been impinged with radiation.

52. Method according to one or more of the preceding claims 26 to 51, **characterised in that** the measuring radiation is coupled into the optic elements used for producing the contact with the test object under a variable specific angle for reaching different interactions of the radiation with the test object.

53. Method according to one or more of the preceding claims 26 to 52, **characterised in that** the radiation returning from the test object is guided to a radiation sensor in the same or in another beam guide element as the measuring radiation impinged on the test object.

54. Method according to one or more of the preceding claims 26 to 53, **characterised in that** the collected measuring signal of the radiation reflected by the test object is transferred into digital form and then statistic errors of measurement are removed, respectively their influence is reduced by suitable measures.

55. Method according to one or more of the preceding claims 26 to 54, **characterised in that** the measuring signal from the content which has to be defined is separated from the measuring signal of other contents, and the nature of the content which has to be defined and thus its presence is proved unambiguously.

## Revendications

1. Appareil de mesure destiné à la détermination de la concentration de substances présentes ou dissoutes dans un liquide, par exemple du sang, consistant en au moins une source de rayonnement prévue pour la production d'un rayonnement, en un capteur ou détecteur de rayonnement, ce détecteur étant situé dans un dispositif optique construit comme un élément d'ATR (attenuated total reflection), **caractérisé en ce que** le dispositif optique construit comme un élément d'ATR comporte un empilement de matériaux optiques d'index de réfraction différents et **en ce que** les couches ayant des index de réfraction différents sont situées à une certaine distance les unes par rapport aux autres.

2. Appareil de mesure selon la revendication 1, **caractérisé en ce que** l'élément d'ATR possède des couches de surface d'épaisseurs différentes.

3. Appareil de mesure selon l'une ou les deux revendications précédentes, **caractérisé en ce que** l'élément d'ATR possède une forme de demi-sphère.

4. Appareil de mesure selon une ou plusieurs des revendications précédentes, **caractérisé en ce que** le matériau de la demi-sphère et celui de la couche extérieure de résonance possèdent un index de réfraction élevé n1 et n3 et celui de la couche de distance intermédiaire possède un index de réfraction n2 plus faible, en utilisant n1 = n3 et n2 < n1 et donc n2 < n3.

5. Appareil de mesure selon une ou plusieurs des revendications précédentes, **caractérisé en ce que** la source de rayonnement est construite de façon à produire un rayonnement cohérent, par exemple une source de rayonnement laser.

6. Appareil de mesure selon une ou plusieurs des revendications précédentes, **caractérisé en ce que** la source de rayonnement est construite comme une source de rayonnement incohérent, par exemple une source de rayonnement de Nernst.

7. Appareil de mesure selon une ou plusieurs des revendications précédentes, **caractérisé en ce qu'**un dispositif de modification ou de limitation de la fréquence de rayonnement est situé au niveau de la source de rayonnement.

8. Appareil de mesure selon une ou plusieurs des revendications précédentes, **caractérisé en ce que** le dispositif modifiant ou limitant la fréquence du rayonnement est un filtre de type passe-bande.

9. Appareil de mesure selon une ou plusieurs des revendications précédentes, **caractérisé en ce qu'**un dispositif assurant une densité d'énergie constante et dépendante de la fréquence est soit situé au niveau de la source de rayonnement soit liée à cette source de rayonnement.

10. Appareil de mesure selon une ou plusieurs des revendications précédentes, **caractérisé en ce que** l'appareil possède au moins un dispositif de modulation de rayonnement.

11. Appareil de mesure selon une ou plusieurs des revendications précédentes, **caractérisé en ce qu'**il existe une source de rayonnement cohérente dont la fréquence peut être modifiée et/ou **en ce qu'**il existe plusieurs sources de rayonnement de domaines différents de longueur d'onde.

12. Appareil de mesure selon une ou plusieurs des revendications précédentes, **caractérisé en ce qu'**il existe une source de rayonnement incohérente ayant une large bande spectrale d'émission.

13. Appareil de mesure selon une ou plusieurs des revendications précédentes, **caractérisé en ce qu'**un guide de rayonnement fait partie de l'appareil assurant une profondeur de pénétration suffisante du rayon de mesure dans des substances à mesurer avec une interaction maximale et une réflexion totale.

14. Appareil de mesure selon une ou plusieurs des revendications précédentes, **caractérisé en ce que** le guide possède une surface d'appui plane ou adaptée au site de la mesure.

15. Appareil de mesure selon une ou plusieurs des revendications précédentes, **caractérisé en ce que** la surface d'appui adaptée ou plane est située à l'extrémité d'un câble de mesure mince.

16. Appareil de mesure selon une ou plusieurs des revendications précédentes, **caractérisé en ce que** des guides d'ondes optiques sont placés entre le laser et l'élément d'ATR et entre l'élément d'ATR et le détecteur de rayonnement.

17. Appareil de mesure selon une ou plusieurs des revendications précédentes, **caractérisé en ce qu'**un guide d'ondes optiques est placé entre le laser et l'élément d'ATR et entre l'élément d'ATR et le détecteur de rayonnement, lequel guide aussi bien le rayonnement émis que le rayonnement à mesurer.

18. Appareil de mesure selon une ou plusieurs des revendications précédentes, **caractérisé en ce que** le guide d'ondes optiques consiste en une fibre cristalline et possède des extrémités de forme hyperbolique afin de diviser le chemin du rayonnement.

19. Appareil de mesure selon une ou plusieurs des revendications précédentes, **caractérisé par** un dispositif saisissant, analysant et enregistrant les données de mesure.

20. Appareil de mesure selon une ou plusieurs des revendications précédentes, **caractérisé en ce que** le système d'analyse des données comporte une mémoire pour stocker des valeurs.

21. Appareil de mesure selon une ou plusieurs des revendications précédentes, **caractérisé par** un système permettant la conversion en valeurs numériques.

22. Appareil de mesure selon une ou plusieurs des revendications précédentes, **caractérisé en ce qu'**une combinaison de plusieurs filtres élémentaires est située entre le guide d'ondes optiques et l'élément d'ATR et entre le guide d'ondes optiques et le détecteur de rayonnement.

23. Appareil de mesure selon une ou plusieurs des revendications précédentes, **caractérisé en ce qu'**un détecteur est placé de telle façon qu'il détecte le degré de polarisation à l'orientation souhaitée après interaction du rayonnement avec l'objet à mesurer.

24. Appareil de mesure selon une ou plusieurs des revendications précédentes, **caractérisé en ce que** des coupleurs sont placés aussi bien au niveau de l'élément d'ATR qu'au niveau du capteur de rayonnement qui permet de coupler des rayonnements entrants et sortants avec le guide d'onde.

25. Appareil de mesure selon une ou plusieurs des revendications précédentes, **caractérisé en ce qu'**il existe un dispositif permettant de pulser le rayonnement pour la mesure.

26. Procédé d'analyse de la concentration de substances en suspension ou dissoutes dans un liquide, par exemple du sang, utilisant un rayonnement émis par le système de mesure dans un liquide à analyser soit in vivo soit ex vivo soit in vitro dont la partie réfléchie est reçue par un système de réception et transmis à un système d'analyse, **caractérisé en ce qu'**un appareil de mesure est utilisé selon une ou plusieurs des revendications précédentes 1 à 25.

27. Procédé selon la revendication précédente 26, **caractérisé en ce que** les données reçues sont comparées aux données stockées dans les systèmes de réception et d'analyse en prenant en compte un modèle mémorisé dans le système d'analyse, basé sur des données sur les influences variables dans l'espace des couches individuelles d'un modèle de tissus en fonction de la longueur d'onde et de la polarisation afin de déterminer l'existence de certaines substances dans le liquide à analyser.

28. Procédé selon l'une ou les deux revendications précédentes 26 et 27, **caractérisé en ce que** la confirmation de l'existence de certaines substances est effectuée à l'aide de méthodes comme l'analyse des clusters, l'analyse discriminatoire ou l'analyse de variance.

29. Procédé selon une ou plusieurs des revendications précédentes 26 à 28, **caractérisé en ce que** ce procédé peut être répété aussi souvent que souhaité.

30. Procédé selon une ou plusieurs des revendications précédentes 26 à 29, **caractérisé en ce que** le rayonnement de mesure émis ainsi que le rayonnement reçu passent par au moins un élément d'ATR et par au moins un élément optique en utilisant des propriétés au moins d' absorption et de réflexion spécifiques aux substances.

31. Procédé selon une ou plusieurs des revendications précédentes 26 à 30, **caractérisé en ce que** le rayonnement de mesure possède une propriété lui permettant de traverser une couche de séparation enveloppant un liquide avant de pénétrer ce liquide et permettant à la partie réfléchie de traverser cette couche avant d'atteindre le détecteur.

32. Procédé selon une ou plusieurs des revendications précédentes 26 à 31, **caractérisé en ce qu'**une substance active sous forme rigide, liquide ou poudreuse est appliquée sur l'émetteur, sur la couche de limitation ou de séparation et sur le détecteur, permettant d'améliorer le couplage du rayonnement de mesure entrant et sortant au niveau du contact entre l'appareil de mesure et l'objet à mesurer.

33. Procédé selon une ou plusieurs des revendications précédentes 26 à 32, **caractérisé en ce que** le rayonnement de mesure est mis en résonance optique à l'aide d'un dispositif optique.

34. Procédé selon une ou plusieurs des revendications précédentes 26 à 33, **caractérisé en ce que** le rayonnement possède une orientation de polarisation unique.

35. Procédé selon une ou plusieurs des revendications précédentes 26 à 34, **caractérisé en ce que** l'orientation du plan de polarisation est modifié de façon à couvrir toutes les orientations possibles en faisant particulièrement attention aux polarisations horizontale et verticale.

36. Procédé selon une ou plusieurs des revendications précédentes 26 à 35, **caractérisé en ce que** des modifications dynamiques supplémentaires sont effectuées lors des modifications des orientations du plan de polarisation, telles des polarisations circulaires ou elliptiques et des polarisations tournant à droite ou à gauche.

37. Procédé selon une ou plusieurs des revendications précédentes 26 à 36, **caractérisé en ce que** l'objet à mesurer est soumis à un rayonnement discontinu tel une pulsation.

38. Procédé selon une ou plusieurs des revendications précédentes 26 à 37, **caractérisé en ce que** le rayonnement de mesure est un rayonnement infrarouge allant de 0,8µm à 100µm de longueur d'onde avec l'infrarouge proche entre 0,8µm et 2,5µm, l'infrarouge intermédiaire entre 2,5µm et 15µm et l'infrarouge lointain entre 15µm et 100µm.

39. Procédé selon une ou plusieurs des revendications précédentes 26 à 38, **caractérisé en ce qu'**une modification et un ajustement de l'énergie du rayonnement sont effectués en fonction de la fréquence et de la polarisation du rayonnement de mesure.

40. Procédé selon une ou plusieurs des revendications précédentes 26 à 39, **caractérisé en ce que** la détermination des coefficients d'absorption dans les trois axes de l'espace d'un échantillon anisotrope est réalisé par une analyse combinatoire des degrés de réflexion pour des orientations de polarisation différentes du rayonnement de mesure.

41. Procédé selon une ou plusieurs des revendications précédentes 26 à 40, **caractérisé par** l'utilisation (l'application) de la méthode des transformations de repliement.

42. Procédé selon une ou plusieurs des revendications précédentes 26 à 41, **caractérisé en ce que** la différenciation des signaux spécifiques aux substances est effectué en filtrant préalablement le ou les signaux.

43. Procédé selon une ou plusieurs des revendications précédentes 26 à 42, **caractérisé en ce que** la cohérence spatiale et temporelle de la source de rayonnement soit prise en compte pour la modélisation.

44. Procédé selon une ou plusieurs des revendications précédentes 26 à 43,**caractérisé par** l'utilisation (application) d'une analyse d'inversion.

45. Procédé selon une ou plusieurs des revendications précédentes 26 à 44, **caractérisé en ce que** les rayonnement de mesure proviennent d'une source cohérente ou incohérente.

46. Procédé selon une ou plusieurs des revendications précédentes 26 à 45, **caractérisé en ce qu'**un effet de sélection ou d'amplification du signal de mesure est obtenu en introduisant préalablement à la mesure une substance réactive dans le liquide provoquant une réaction sous l'effet du rayonnement de mesure ou d'un autre type de rayonnement résultant en une modification temporaire ou permanente de la signature des rayonnements émis par le liquide ou par ses composants.

47. Procédé selon une ou plusieurs des revendications précédentes 26 à 46, **caractérisé en ce que** dans le cas de détection de concentrations extrêmement faibles, un composé réactif est introduit dans le liquide afin d'amplifier la réaction provoquée de sorte qu'elle permet d'obtenir des signaux dépassant les niveaux stochastiques et le bruit systématique.

48. Procédé selon une ou plusieurs des revendications précédentes 26 à 47, **caractérisé en ce qu'**une substance active est appliquée sur la parois extérieure de la couche de séparation faisant face au détecteur ou sur la face extérieure du détecteur provoquant une atténuation ou amplification sélective et ainsi une construction significative d'une signature caractéristique nécessaire pour la détection d'une substance ou d'une réaction.

49. Procédé selon une ou plusieurs des revendications précédentes 26 à 48, **caractérisé en ce que** l'entrée du rayonnement dans l'objet à mesurer s'effectue à l'endroit sur l'objet ou s'effectue la sortie.

50. Procédé selon une ou plusieurs des revendications précédentes 26 à 49, **caractérisé en ce que** seule une certaine bande des fréquences du rayonnement de mesure parmi la gamme de longueurs d'ondes disponibles est utilisée et déplacée continuellement à l'intérieur d'une gamme de longueurs d'ondes nécessaires pour la détermination des substances spécifiques et **en ce que** ce déplacement est effectué une ou plusieurs fois durant le temps de mesure en modifiant l'énergie en fonction de la longueur d'onde du rayonnement.

51. Procédé selon une ou plusieurs des revendications précédentes 26 à 50, **caractérisé en ce que** la modification du rayonnement de mesure est non seulement effectuée avant l'exposition de l'objet au niveau du rayonnement entre la source et l'objet à mesurer, mais également après l'exposition de l'objet entre l'objet à mesurer et le détecteur.

52. Procédé selon une ou plusieurs des revendications précédentes 26 à 51, **caractérisé en ce que** le rayonnement de mesure rentre sous un angle modifiable dans les éléments optiques nécessaires pour la réalisation du contact avec l'objet à mesurer afin d'obtenir des interactions différentes entre le rayonnement et l'objet à mesurer.

53. Procédé selon une ou plusieurs des revendications précédentes 26 à 52, **caractérisé en ce que** le rayonnement venant de l'objet à mesurer est guidé par le même ou par un autre guide d'onde vers le détecteur que celui guidant le rayonnement arrivant sur l'objet.

54. Procédé selon une ou plusieurs des revendications précédentes 26 à 53, **caractérisé en ce que** le signal mesuré du rayonnement réfléchi par l'objet à mesurer est transformé en un signal numérique dont les erreurs de mesure systématiques ou statistiques sont diminuées ou enlevées.

55. Procédé selon une ou plusieurs des revendications précédentes 26 à 54, **caractérisé en ce que** le signal de mesure d'une substance à déterminer est séparé de celui des autres substances ce qui permet de déterminer sans équivoque l'origine de la substance et donc son existence.
